(19)

Europäisches
Patentamt
European
Patent Office
Office européen
des brevets

(11) **EP 2 103 944 A1**

(12) **EUROPEAN PATENT APPLICATION**

(43) Date of publication:
**23.09.2009 Bulletin 2009/39**

(51) Int Cl.:
***G01N 33/68*** *(2006.01)*

(21) Application number: **08290264.4**

(22) Date of filing: **20.03.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicant: **sanofi-aventis**
**75013 Paris (FR)**

(72) Inventor: **The designation of the inventor has not yet been filed**

(74) Representative: **Schneider, Rudolf et al**
**Sanofi-Aventis Deutschland GmbH**
**Patente Deutschland**
**Industriepark Höchst**
**Gebäude K 801**
**D-65926 Frankfurt am Main (DE)**

(54) **Fluorescence based assay to detect sodium/calcium exchanger "forward mode" modulating compounds**

(57)  Transporters are an emerging target family with enormous potential, offering scientific and economic opportunities. The sodium/calcium exchanger is an important mechanism for removing $Ca^{2+}$ from diverse cells. In heart, it extrudes $Ca^{2+}$ that has entered through $Ca^{2+}$ channels to initiate contraction, while $Na^+$ enters the heart cell. It is of considerable interest to identify compounds that modulate the activity of sodium/calcium exchangers. The present invention is directed to a fluorescence-based assay for detecting NCX and NCKX "forward mode" modulating compounds. It further refers to a kit of parts comprising cells expriming a sodium/calcium exchanger and the use of the kit of parts to test a compound for activity as an agonist or antagonist of a sodium/calcium exchanger.

EP 2 103 944 A1

**Description**

FIELD OF THE INVENTION

The present invention relates to sodium/calcium exchangers and methods for determining their activity. More specifically, the invention relates to a fluorescence-based assay for detecting sodium/calcium exchanger "forward mode" modulating compounds. It further refers to a kit of parts comprising cells expressing a sodium/calcium exchanger and the use of the kit of parts.

BACKGROUND OF THE INVENTION

A basic requirement for life is compartmentalization - with biological membranes being nature's tool to realize this principle. However, a lipid bilayer - the structure underlying the cell membrane - is impermeable to most ions and compounds whose transport is essential to sustain vital functions in cells and organisms. The answer to this paradox lies in the semipermeable nature of the cell membrane - solutes that have to cross the membrane are transported by specific membrane proteins. These transporters are responsible for the generation and maintenance of ion gradients, the uptake of nutrients, the transport of metabolites, the reuptake of signaling molecules and the disposal of toxic and waste compounds. Therefore, transporters are potential drug targets that allow direct influence on disease-related abnormalities in this context.

The sodium/calcium exchanger human gene family (also known as NCX or SLC8) encompasses three distinct proteins, NCX1, NCX2 and NCX3. SLC8 together with SLC24 constitute a superfamily of $Na^+/Ca^{2+}$ countertransporters. SLC24 family members also transport $K^+$, they are also known as NCKXs. NCX1 is the most highly characterized member of this family, its expression is up regulated in failing human heart and is involved in ischemia-reperfusion damage after myocardial infarction. Inhibition of NCX1 normalizes heart muscle contractility in failing hearts and acts and Ohtsuka, Journal of Pharmacological. Sciences. 2004). NCX2 is mainly expressed in the brain and NCX3 in the brain and skeletal muscle, their physiological roles remain elusive.

The sodium/calcium exchanger is an important mechanism for removing $Ca^{2+}$ from diverse cells. In heart, it extrudes $Ca^{2+}$ that has entered through $Ca^{2+}$ channels to initiate contraction, while $Na^+$ enters the heart cell. Its relevance in cardiovascular diseases is e.g. illustrated in Hobai, JA & O'Rourke, B (2004) Expert Opin. Investig. Drugs, 13, 653-664. Therefore, pharmaceutical industry has developed compounds inhibiting the NCX as e.g. described in Iwamoto, T. et al. (2004) J. Biol. Chem., 279, 7544-7553. The $Na^+/Ca^{2+}$ exchanger electrogenically transports three to four $Na^+$ for every $Ca^{2+}$ that moves in the opposite direction as e.g. shown by electrophysiological means in Hinata, M. et al. (2002) J. Physiol. 545, 453-461. The NCX is able to maintain the cytoplasmic $Ca^{2+}$ concentration ($[Ca^{2+}]$ in) three to four orders of magnitude below the extracellular $Ca^{2+}$ concentration ($[Ca^{2+}]$ out). Nevertheless, the direction of net $Ca^{2+}$ transport depends on the electrochemical gradient of $Na^+$. Simultaneous and consecutive transport models have been suggested for $Na^+$ and $Ca^{2+}$ translocations, and a bulk of evidence favors the latter.

Transporters are an emerging target family with enormous potential, offering scientific and economic opportunities. On the other hand, transporters are a difficult target class in terms of drug-discovery technologies.

It is of considerable interest to identify compounds that modulate channel activity, for example, by blocking the flow of calcium and/or inhibiting the activation of calcium channels. One standard method to do so is through the use of patch clamp experiments.

In these experiments, cells must be evaluated individually and in sequence by highly skilled operators, by measuring the calcium current across the cell membrane in response to changes of the membrane potential and/or application of test compounds. The effect of Sea0400, a new specific inhibitor of NCX, on the action potential in dog ventricular papillary muscle was investigated and disclosed by K. Acsai during the "ESC Congress 2004" in Munich on Poster Nr. 2886 (Title: Effect of a specific sodium-calcium exchanger blocker Sea0400 on the ventricular action potential and triggered activity in dog ventricular muscle and Purkinje fiber) and by C. Lee et al. (The journal of pharmacology and experimental therapeutics; Vol. 311: 748-757, 2004; Title: Inhibitory profile of SEA0400 [2-[4-[(2,5-Difluorophenyl)methoxy]phenoxy]-5-ethoxyaniline] assessed on the cardiac $Na^+/Ca^{2+}$ exchanger, NCX1.1). It was shown, using an ion-selective electrode technique to quantify ion fluxes in giant patches, that the cardiac $Na^+/Ca^{2+}$ exchanger has multiple transport modes (Tong Mook Kang & Donald W. Hilgemann; Nature; Vol. 427, 5 February 2004; Title: Multiple transport modes of the cardiac $Na^+/Ca^{2+}$ exchanger).

These experiments, while valid and informative, are very time consuming and not adaptable to high-throughput assays for compounds that modulate calcium ion channel activity.

Various techniques have been developed as alternatives to standard methods of electrophysiology. For example, radioactive flux assays have been used in which cells are exposed with a radioactive tracer (e.g., $^{45}Ca$) and the flux of the radio-labled Ca is monitored. Cells loaded with the tracer are exposed to compounds and those compounds that either enhance or diminish the efflux of the tracer are identified as possible activators or inhibitors of ion channels in the cells' membranes. A specific example is enclosed in T. Kuramochi et al.; Bioorganic & Medicinal Chemistry; 12 (2004) 5039-5056; Title: Synthesis and structure-activity relationships of phenoxypyridine derivates as novel inhibitors of the sodium-calcium exchanger. EP1031556 discloses a method wherein $Na^+/Ca^{2+}$ exchanger activity is measured using sarcolemmal vesicles, the concentration of $Ca^{2+}$ uptake in the sarcolemmal vesicles being determined by measuring $^{45}Ca$ radioactivity.

Many radioactive ion-transporter assays have limited sensitivity and therefore insufficient date quality. In addition, the cost and safety issues associated with the radioactive screening technology are hurdles that hinder a broadened application.

Among the above cited drug-discovery technologies, the use of radioactive flux assays to identify compounds that modulate the activity of ion channels and ion transporters is the closest prior art to our invention as it is a technique in which a test compound can be identified as possible activator or inhibitor by monitoring the flux of $Ca^{2+}$ from the cells. The main issue for the radioactive assays is based on the difficulty of detecting the limited turnover of ion transporters of about 1 to 1000 molecules per second - about $10^4$ times less than most ion channels.

The problem arising from the state of the art therefore was to identify a robust assay with a very good sensitivity and usefulness for high throughput screening and profiling of sodium/calcium exchanger modulators. The solution of that problem is provided by the present invention.

SUMMARY OF THE INVENTION

One subject-matter of the present invention refers to an assay for determining the activity of a sodium/calcium exchanger wherein:

> a) cells expressing a sodium/calcium exchanger are provided;
> b) a colored substance for determining intracellular calcium is provided;
> c) cells are contacted with a sodium/calcium exchanger activator; and
> d) the calcium mediated change in the luminescent signal from said colored substance is compared to a luminescent signal produced in a control experiment.

Another subject-matter of the present invention refers to an assay for determining the activity of a sodium/ calcium exchanger in response to the addition of a compound wherein:

> a) cells expressing a sodium/calcium exchanger are provided;
> b) a colored substance for determining intracellular calcium is provided;
> c) cells are contacted with a compound, wherein said cells have been treated, prior to treating with said compound, with a sodium/calcium exchanger activator; and
> d) the calcium mediated change in the luminescent signal from said colored substance is compared to a luminescent signal produced in a control experiment.

In general, the sodium/calcium exchanger used was of mammalian origin, and in particular of human origin. The sodium/calcium exchanger is selected from one of the following sodium/calcium exchanger proteins: NCX1, NCX2, NCX3, NCX4, NCX5, NCX6 and/or NCX7, in particular NCX1, NCX2 and/or NCX3; and/or from one of the following sodium/calcium/potassium exchanger proteins: NCKX1, NCKX2, NCKX3, NCKX4 and/or NCKX5.

In general, the cells used in the assay of the present invention can be derived from any eukaryotic organism. In a preferred embodiment, the cells are mammalian cells. In a more preferred embodiment, the cells are CHO (CCL-61), HEK (CCL-1573), COS7 (CRL-1651) and/or JURKAT (CRL-1990) cells.

In particular, the sodium/calcium exchanger activator used in the assay of the present invention is ionomycin.

In a preferred embodiment, said colored substance is added to the cells as a dye precursor capable of entering the cells and being hydrolyzed to a dye, whereby the dye complexes with calcium in said cells and provides a luminescent signal. Further said dye precursor can be preferably an acetoxymethylester derivate and said dye can be preferably the calcium sensitive fluorescence dye fluo-4. In a more preferred embodiment, said luminescent signal is fluorescence and said monitoring step c) employs a FLIPR device.

The invention pertains further to the use of an assay as mentioned before to test a compound for activity as an agonist or antagonist of a sodium/calcium exchanger. In another preferred embodiment, the invention pertains to the use of an assay as mentioned before for the diagnosis of a disease associated with a sodium/calcium exchanger altered expression.

The invention pertains further to a kit of parts comprising:

> a) lyophilized cells expriming a sodium/calcium exchanger;

b) a colored substance;

c) a compound buffer; and

d) a colored substance buffer.

In a preferred embodiment of the kit of parts of the present invention, said colored substance is the calcium sensitive fluorescence dye fluo-4. In another preferred embodiment, the sodium/calcium exchanger used was of mammalian origin, and in particular of human origin. The sodium/calcium exchanger is selected from one of the following sodium/calcium exchanger proteins: NCX1, NCX2, NCX3, NCX4, NCX5, NCX6 and/or NCX7, in particular NCX1, NCX2 and/or NCX3; and/or from one of the following sodium/calcium/potassium exchanger proteins: NCKX1, NCKX2, NCKX3, NCKX4 and/or NCKX5.

The invention pertains further to the use of a kit of parts as mentioned before to test a compound for activity as an agonist or antagonist of a sodium/calcium exchanger. In another preferred embodiment, the invention pertains to the use of a kit of parts as mentioned before for the diagnosis of a disease associated with a sodium/calcium exchanger altered expression.

DETAILED DESCRIPTION OF THE INVENTION

The term "assay" refers to a procedure where a property of a system or object is measured.

Assay is a short hand commonly used term for biological assay and is a type of in vitro experiment. Assays are typically conducted to measure the effects of a substance on a living organism. Assays may be qualitative or quantitative, they are essential in the development of new drugs.

The subject assay provides a broad dynamic range so that the activity of a sodium/calcium exchanger can be determined. In particular the present invention makes available a rapid, effective assay for screening and profiling pharmaceutically effective compounds that specifically interact with and modulate the activity of a sodium/calcium exchanger.

The term " sodium/calcium exchanger " or "NCX" in context of the present invention shall mean any one of the list of the following $Na^+/Ca^{2+}$ exchanger proteins: NCX1, NCX2, NCX3, NCX4, NCX5, NCX6, NCX7; or any one of the list of the following $Na^+/Ca^{2+}/K^+$ exchanger proteins: NCKX1, NCKX2, NCKX3, NCKX4, NCKX5, either alone or in combination with each other.

Especially preferred are the SLC8 family members NCX1, NCX2 and/or NCX3 which amino acid sequences correspond, respectively, to SEQ ID NO: 1, SEQ ID NO: 2 and SEQ ID NO: 3.

Such a sodium/calcium exchanger could be derived from any vertebrate and in particular mammalian species (e.g. dog, horse, bovine, mouse, rat, canine, rabbit, chicken, anthropoid, human or others). The sodium/calcium exchanger could be isolated from tissue probes of such vertebrate organisms or could be manufactured by means of recombinant biological material that is able to express the sodium/calcium exchanger.

The term "sodium/calcium exchanger protein" refers to polypeptides, polymorphic variants, mutants, and interspecies homologues that have an amino acid sequence that has greater than about 80% amino acid sequence identity, 85%, 90%, preferably 91%, 92%, 93%, 94%, 95%, 96%, 97%, 98% or 99% or greater amino acid sequence identity, preferably over a region of at least about 25, 50, 100, 200, or 500, or more amino acids, to an amino acid sequence contained in SEQ ID NO: 1, SEQ ID NO: 2 and SEQ ID NO: 3.

The term "biological material" means any material containing genetic information and capable of reproducing itself or being reproduced in a biological system. Recombinant biological material is any biological material that was produced, has been changed or modified by means of recombinant techniques well known to a person skilled in the art.

The following references are examples of the cloning of particular NCX proteins: The canine $Na^+/Ca^{2+}$ exchanger NCX1 has been cloned by Nicoll, DA. et al. (Science. 250(4980): 562-5, 1990; Title: Molecular cloning and functional expression of the cardiac sarcolemmal Na(+)-Ca2+ exchanger.). The human $Na^+/Ca^{2+}$ exchanger NCX1 has been cloned by Komuro, I., et al. (Proc. Natl. Acad. Sci. U.S.A. 89 (10), 4769- 4773, 1992; Title: Molecular cloning and characterization of the human cardiac $Na^+/Ca^{2+}$ exchanger cDNA) and by Kofuji, P. et al. (Am. J. Physiol. 263 (Cell Physiol. 32): C1241-C1249, 1992; Title: Expression of the Na-Ca exchanger in diverse tissues: a study using the cloned human cardiac Na-Ca exchanger). The human $Na^+/Ca^{2+}$ exchanger NCX2 has been cloned by Li, Z. et al. (J. Biol. Chem. 269(26): 17434-9, 1994; Title: Cloning of the NCX2 isoform of the plasma membrane Na(+)-Ca2+ exchanger). The rat $Na^+/Ca^{2+}$ exchanger NCX3 has been cloned by Nicoll, DA. et. al. (J. Biol. Chem. 271 (40): 24914-21. 1996; Title: Cloning of a third mammalian $Na^+/Ca^{2+}$ exchanger, NCX3). The human $Na^+/Ca^{2+}$ exchanger NCX3 has been cloned by Gabellini, N. et. al. (Gene. 298: 1-7, 2002; Title: The human SLC8A3 gene and the tissue-specific $Na^+/Ca^{2+}$ exchanger 3 isoforms).

The terms "polypeptide", "peptide" and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymer.

The term "activity of a sodium/calcium exchanger" refers to the mechanism of removing intracellular $Ca^{2+}$ from a cell. In heart, it extrudes $Ca^{2+}$ that has entered through $Ca^{2+}$ channels to initiate contraction, while $Na^+$ enters the heart cell. Its relevance in cardiovascular diseases is e.g. illustrated in Hobai, JA & O'Rourke,B (2004) Expert Opin. Investig. Drugs, 13, 653-664. Therefore, pharmaceutical industry has developed compounds inhibiting the NCX as e.g. described in Iwamoto, T. et al. (2004) J. Biol. Chem., 279, 7544-7553. The $Na^+/Ca^{2+}$ exchanger electrogenically transports three to four $Na^+$ for every $Ca^{2+}$ that moves in the opposite direction as e.g. shown by electrophysiological means in Hinata, M. et al. (2002) J. Physiol. 545, 453-461. The NCX is able to maintain the cytoplasmic $Ca^{2+}$ concentration ($[Ca^{2+}]$ in) three to four orders of magnitude below the extracellular $Ca^{2+}$ concentration ($[Ca^{2+}]$ out). Nevertheless, the direction of net $Ca^{2+}$ transport depends on the electrochemical gradient of $Na^+$. Simultaneous and consecutive transport models have been suggested for $Na^+$ and $Ca^{2+}$ translocations, and a bulk of evidence favors the latter. The activity of a sodium/calcium exchanger is determined by measuring the enhanced luminescence resulting from a suitable colored substance complexing with calcium.

The term "cells expressing a sodium/calcium exchanger" refers to cells expressing the exchanger of interest endogenously or recombinant cells.

The term "recombinant" when used with reference, e. g., to a cell, or nucleic acid, protein, or vector, indicates that the cell, nucleic acid, protein or vector, has been modified by the introduction of a heterologous nucleic acid or protein or the alteration of a native nucleic acid or protein, or that the cell is derived from a cell so modified. Thus, for example, recombinant cells express genes that are not found within the native (non-recombinant) form of the cell or express native genes that are otherwise abnormally expressed, under expressed or not expressed at all. In the present invention this typically refers to cells that have been transfected with nucleic acid sequences that encode a sodium/calcium exchanger.

The assay is performed simply by growing the cells in an appropriate container with a suitable culture medium. The cell may be a naturally occurring cell, a native cell, an established cell line, a commercially available cell, a genetically modified cell, etc. so long as the cell is able to be maintained during the assay and desirably growing in a culture medium.

Suitable cells for generating the subject assay include prokaryotes, yeast, or higher eukaryotic cells, especially mammalian cells. Prokaryotes include gram negative and gram positive organisms. The cells will usually be mammalian cells, such as human cells, mouse cells, rat cells, Chinese hamster cells, etc. Cells that are found to be convenient include CHO, COS7, JURKAT, HeLa, HEKs, MDCK and HEK293 cells.
Cells may be prepared with the well known methods (Current protocols in cell biology, John Wiley & Sons Inc, ISBN: 0471241059) or may be bought (Invitrogen Corp., Sigma-Aldrich Corp., Stratagene).

The term "colored substance" refers in particular to a calcium sensitive fluorescence dye. The dye precursor is characterized by not being luminescent under the conditions of the assay, being an ester capable of entering the cells and that is hydrolyzed intracellularly to the luminescent oxy compound, and providing enhanced luminescence upon complexing with calcium. The esters are chosen to be susceptible to hydrolysis by intracellular hydrolases.

The term "capable of entering the cells" means that the precursors are able to cross the cellular membrane and be hydrolyzed in the cells, the dye precursor enters the cells under specific conditions of pH, temperature, etc., enters the cells at different speeds or does not enter the cells under specific conditions.

The colored substance is added to the cells using the well known protocols (Current protocols in cell biology, John Wiley & Sons Inc, ISBN: 0471241059).
The use of a colored substance is conventional and commercially available reagents (Invitrogen Corp.) as well as reagents synthesized in laboratory can be used.
A number of commercially available dyes fulfilling the above requirements are known. Fluorescent dyes for monitoring $Ca^{2+}$ are well known and described in detail in section 20.1-20.4 of the Molecular Probes catalog, 9th edition. They usually have two bis-carboxymethylamino groups attached to a fluorescent nucleus such as fluoresceins, rhodamines, coumarins, aminophenylindoles, and others. For the most part the compounds are 3,6-dioxy substituted xanthenes, where in the precursor the oxy groups are substituted and in the luminescent dye they are unsubstituted. Usually there are acetoxymethyl groups protecting the phenols and acids. See, for example, Fluo3/4, Fura2/3, calcein green, etc. Hydrolysis of the acetyl group results in the luminescent product. The precursors are able to cross the cellular membrane and be hydrolyzed in the cell.

The term "luminescence" refers to a "cold light", light from other sources of energy, which can take place at normal and lower temperatures. In luminescence, some energy source kicks an electron of an atom out of its "ground" (lowest-energy) state into an "excited" (higher-energy) state; then the electron gives back the energy in the form of light so it can fall back to its "ground" state. There are several varieties of luminescence, each named according to what the source of energy is, or what the trigger for the luminescence is.

The term "fluorescence" refers to a luminescence that is mostly found as an optical phenomenon in cold bodies, in which the molecular absorption of a photon triggers the emission of another photon with a longer wavelength. The energy difference between the absorbed and emitted photons ends up as molecular vibrations or heat. Usually the absorbed photon is in the ultraviolet range, and the emitted light is in the visible range, but this depends on the absorbance curve and Stokes shift of the particular fluorophore. Fluorescence is named after the mineral fluorite, composed of calcium fluoride, which often exhibits this phenomenon.

Fluorescence from the indicator dyes can be measured with a luminometer or a fluorescence imager. One preferred detection instrument is the Fluorometric Imaging Plate Reader (FLIPR) (Molecular Devices, Sunnyvale, Calif.). The FLIPR is well suited to high throughput screening using the methods of the present invention as it incorporates integrated liquid handling capable of simultaneously pipetting to 96 or 384 wells of a microtiter plate and rapid kinetic detection using a argon laser coupled to a charge-coupled device imaging camera.

An alternative to the use of calcium indicator dyes is the use of the aequorin system. The aequorin system makes use of the protein apoaequorin, which binds to the lipophilic chromophore coelenterazine forming a combination of apoaequorin and coelenterazine that is known as aequorin. Apoaequorin has three calcium binding sites and, upon calcium binding, the apoaequorin portion of aequorin changes its conformation. This change in conformation causes coelenterazine to be oxidized into coelenteramide, $CO_2$, and a photon of blue light (466 nm). This photon can be detected with suitable instrumentation. For reviews on the use of aequorin, see Créton et al., 1999, Microscopy Research and Technique 46:390-397; Brini et al., 1995, J. Biol. Chem. 270:9896-9903; Knight & Knight, 1995, Meth. Cell. Biol. 49: 201-216. Also of interest may be U.S. Pat. No. 5,714,666 which describes methods of measuring intracellular calcium in mammalian cells by the addition of coelenterazine co-factors to mammalian cells that express apoaequorin.

"Inhibitors" are compounds that, e. g., bind to, partially or totally block activity, decrease, prevent, delay activation, inactivate, desensitize, or down regulate the activity or expression of sodium/calcium exchanger proteins, e. g., antagonists. "Activators" are compounds that increase, open, activate, facilitate, enhance activation, sensitize, agonize, or up regulate sodium/calcium exchanger activity. A prefered sodium/calcium exchanger activator is ionomycin, an ionophore that comes from Streptomyces conglobatus.

Inhibitors, activators, or modulators also include genetically modified versions of sodium/calcium exchanger proteins, e. g., versions with altered activity, as well as naturally occurring and synthetic ligands, antagonists, agonists, peptides, cyclic peptides, nucleic acids, antibodies, antisense molecules, ribozymes, small organic molecules and the like.

The term "compound" or "test compound" or "test candidate" or grammatical equivalents thereof describes any molecule, either naturally occurring or synthetic, e. g., protein, oligopeptide, small organic molecule, polysaccharide, lipid, fatty acid, polynucleotide, oligonucleotide, etc., to be tested for the capacity to modulate sodium/calcium exchanger activity (Current protocols in molecular biology, John Wiley & Sons Inc, ISBN: 0471250961). The test compound can be in the form of a library of test compounds, such as a combinatorial or randomized library that provides a sufficient range of diversity (Current protocols in molecular biology, John Wiley & Sons Inc, ISBN: 0471250937). Test compounds are optionally linked to a fusion partner, e. g., targeting compounds, rescue compounds, dimerization compounds, stabilizing compounds, addressable compounds, and other functional moieties. Conventionally, new chemical entities with useful properties are generated by identifying a test compound (called a "lead compound") with some desirable property or activity, e. g., enhancing activity, creating variants of the lead compound, and evaluating the property and activity of those variant compounds. Preferably, high throughput screening (HTS) methods are employed for such an analysis.

Said inhibitor, activator and test compound may be added to the cells by injection into the culture medium after the cells have grown or they may be present in the culture medium prior to the cell growth (Current protocols in cell biology, John Wiley & Sons Inc, ISBN: 0471241059).

The cells may be grown to the appropriate number on the inhibitor, activator and/or test compound or they may be placed on it and used without further growth. The cells may be attached to the inhibitor, activator and/or test compound or, in those embodiments where the cells are placed or grown in wells, the cells may be suspension cells that are suspended in the fluid in the wells.

The term "control experiment" refers to different kinds of experiments that should be run together. The skilled person will recognize that it is generally beneficial to run controls together with the methods described herein.

For example, it will usually be helpful to have a control for the assay for determining the activity of a sodium/calcium exchanger in which the cells are preferably essentially identical to the cells that are used in the assay except that these cells would not express the sodium/calcium exchanger of interest.

Furthermore, it will usually be helpful to have a control for the assay for determining the activity of a sodium/calcium exchanger in response to the addition of a compound in which the compounds are tested in the assay of the invention against cells that preferably are essentially identical to the cells that are used in the assay except that these cells would not express the sodium/calcium exchanger of interest. In this way it can be determined that compounds which are identified by the assay are really exerting their effects through the sodium/calcium exchanger of interest rather than through some unexpected non-specific mechanism. One possibility for such control cells would be to use non-recombinant parent cells where the cells of the actual experiment express the sodium/calcium exchanger of interest. Other controls for the assay for determining the activity of sodium/calcium exchanger in response to the addition of a compound would be to run the assay without adding a test compound (low control) and to run the assay with a high concentration of test compound (high control).

Other types of controls would involve taking compounds that are identified by the assay of the present invention as agonists or antagonists of sodium/calcium exchangers of interest and testing those compounds in the methods of the prior art in order to confirm that those compounds are also agonists or antagonists when tested in those prior art methods. Furthermore, one skilled in the art would know that it also desirable to run statistical analysis by comparing the assay values to standard values.

The terms "agonist" and "antagonist" refer to receptor effector molecules that modulate signal transduction via a receptor. Receptor effector molecules are capable of binding to the receptor, though not necessarily at the binding site of the natural ligand. Receptor effectors can modulate signal transduction when used alone, i.e. can be surrogate ligands, or can alter signal transduction in the presence of the natural ligand, either to enhance or inhibit signaling by the natural ligand. For example, "antagonists" are molecules that block or decrease the signal transduction activity of receptor, e.g., they can competitively, noncompetitively, and/or allosterically inhibit signal transduction from the receptor, whereas "agonists" potentiate, induce or otherwise enhance the signal transduction activity of a receptor.

The term "disease associated with a sodium/calcium exchanger altered expression" refers to dilated cardiomyopathy, coronary heart disease, arrhythmia, heart failure, etc.

For convenience, the colored substance and other components of the assay may be provided in kits, where the colored substance may be present as a reconstitutable powder or as a cooled solution on ice, in a buffer. The kit may also include buffer, activator, inhibitor, test compound, cells expriming a sodium/calcium exchanger protein, etc.. Cells may be present as lyoplilized cells.

Said kit of parts can be used as a diagnostic kit for diagnosing dilated cardiomyopathy, coronary heart disease, arrhythmia, heart failure, etc.

The following figures and examples shall describe the invention in further details, describing the typical results of the fluorescence based cellular sodium/calcium exchanger assay, without limiting the scope of protection.

EXEMPLIFICATION

**1. Assay procedure**

**1.1. Assay reagents**

The following chemical compositions are used as reagents for the assay:

| Reagent | Chemicals | Remarks |
|---|---|---|
| Assay buffer | 3.5 mM $CaCl_2$<br>133.8 mM NaCl<br>4.7 mM KCl<br>1.25 mM $MgCl_2$<br>0.01 % Pluronic F-127<br>10 mM Hepes/NaOH pH 7.5<br>5 mM Glucose<br>2.5 mM Probenecid | Probenecid is added on the day of use from a freshly prepared 1 M solution in 1 N NaOH. |
| Dye loading buffer | Assay buffer containing<br>2 $\mu$M Fluo-4/AM<br>0.1 % BSA | Fluo-4/AM is added from a 1 mM stock solution in DMSO |
| Compound buffer | Assay buffer<br>Various compound concentrations | Compounds are added from a 10 mM stock solution in DMSO |
| Ionophor solution | Assay buffer containing<br>0.3 % BSA<br>6 $\mu$M Ionomycin | Ionomycin is added from a 10 mM stock solution in DMSO |

(continued)

| Reagent | Chemicals | Remarks |
|---|---|---|
| Positive control buffers | low) Ionophor solution | A000135933 is added from a 10 mM stock solution in DMSO |
| | high) Assay buffer 15-45 $\mu$M A000135933 | |

## 1.2. Assay procedure

1] 20-24 h before the experiment, cells are suspended in growth medium (Nutrient Mixture F12 (HAM) Invitrogen, Karlsruhe, 5% FCS, Biochrom, Berlin) without antibiotics and seeded into 96-well black clear bottom plates (25000 cells/well in 100 $\mu$l).

2] Medium is discarded and subsequently 100 $\mu$l of dye loading buffer are added and plates are incubated dark for 75 min at RT.

3] Dye loading buffer is removed by washing three times with 100 $\mu$l assay buffer. Buffer is discarded

4] 80 $\mu$l from compound plates are added and plates are stored for 30 min at 16°C.

5] Plates are transferred into the FLIPR and assayed using the following protocol (including 40 $\mu$l addition from ionophor plate):

| **1.1 FLIPR Experimental Setup Parameters** | |
|---|---|
| Exposure | 0.5 sec (at 1.2 W) |
| F-Stop | F/2 |
| Filter | 1 |
| **1.1.1 Graph Setup** | |
| Subtract Bias Based on Sample: off | |
| Spatial Uniformity Correction: off | |
| Negative Control Correction: off | |
| **1.1.2 First Sequence** | |
| Initial Period | 2 sec |
| Initial Count | 100 frames |
| Add After Frame | 5 |
| Add Height | 70 $\mu$l |
| Add Speed | 40 $\mu$l/sec |
| Add Volume | 40 $\mu$l |
| Mix | 1x40 $\mu$l |
| Statistics | |
| Statistic 1 | sum 25-45 (bias off) |

## 1.3. Data Analysis

Inhibitory Activity of Test Compounds in NCX Cells:

Calculation of inhibition:

Calculations are based on the statistics export. Raw data are converted to inhibition according to:

$$\% - INHIBITION = 100 \times \left( \frac{sample - mean\ low\ control}{mean\ high\ control - mean\ low\ control} \right)$$

Mean high control is derived from the average difference of eight paired samples of 10 or 30 $\mu$M A000135933 with ionomycine. Mean low control is derived from ionomycine controls. Compounds which increases the basal fluorescence higher than 1.3 fold are discarded.

**2. Assay examples**

**2.1. Response of the high and low controls.**

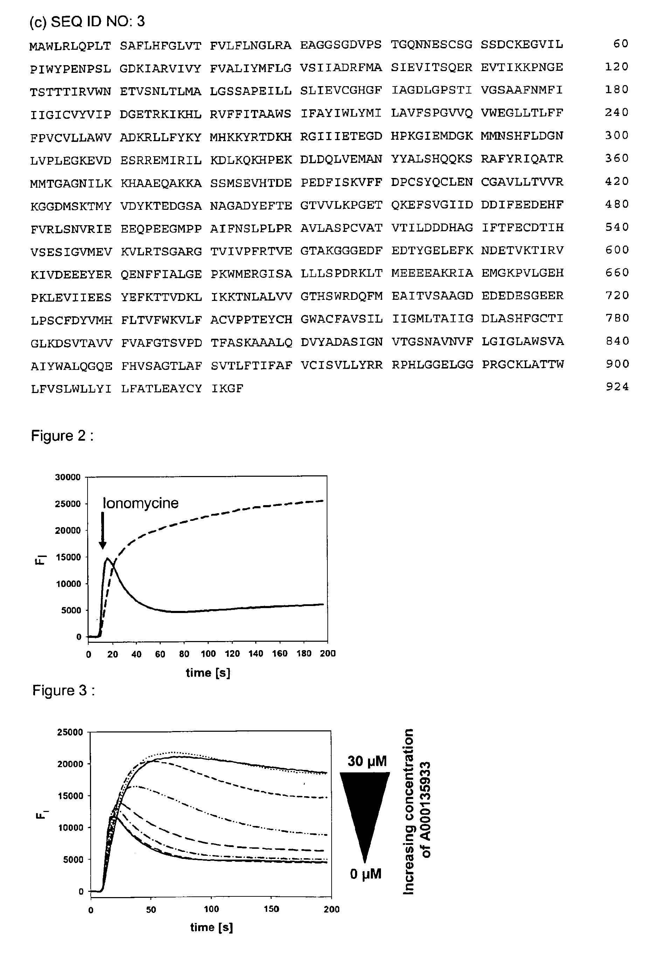

The typical fluorescence response of the high and low controls after addition of 2 $\mu$M Ionomycine is shown in figure 2 and is as following: If the NCX1 is active (low control) calcium entering the cells after Ionomycine addition is transported out of the cells. After a few seconds the initial calcium load of the cells is reestablished. Inhibition of NCX1 leads to a fluorescence increase after Ionomycine addition due to an increase of cytosolic calcium (high control, 30 $\mu$M A000135933).

**2.2. Tool substance: A000135933**

The new NCX1 inhibitor A000135933 was found in the first HTS screen. Figures 3, 4 and 5 show a typical dose dependent response of different concentrations of A000135933. A000135933 was a good NCX1 Inhibitor with a mean $IC_{50}$ of 5.9 $\mu$M and since that time used as tool substance in the assays. An $IC_{50}$ of this compound is added on every plate as control. The S/B ratio and the z' value for this example were very good. Together with the $IC_{50}$ of A000135933 these parameters were used to indicate good assay performance for every plate:

1. S/B greater than two.
2. z' value between 0.5 and 0.7.
3. $IC_{50}$ of the tool compound A000135933 has to be around the mean of 5.9 $\mu$M.

**2.3. Tool substance: Assay example**

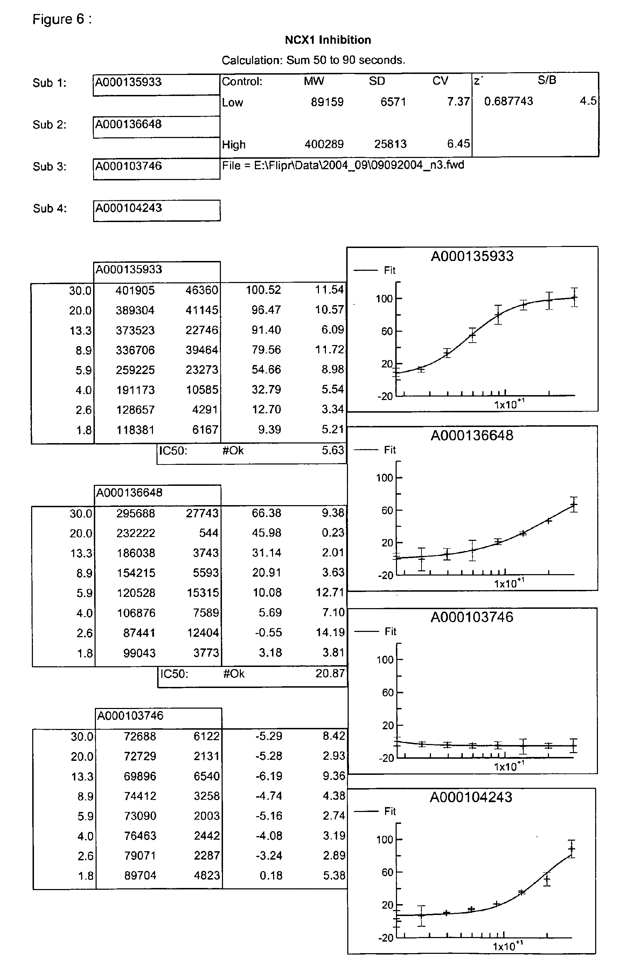

An assay was performed with four compounds $IC_{50}$s in duplicate (Figure 6). The four compounds are from the same compound class. One compound was a good NCX1 inhibitor (A000135933), two compounds show moderate inhibition (A000136648, A000104243) and one was not active in the concentration range (A000103746). This example indicates that the assay is suitable so screen NCX1 inhibitors and to establish structure activity relationships.

**2.4. Correlation with electrophysiology**

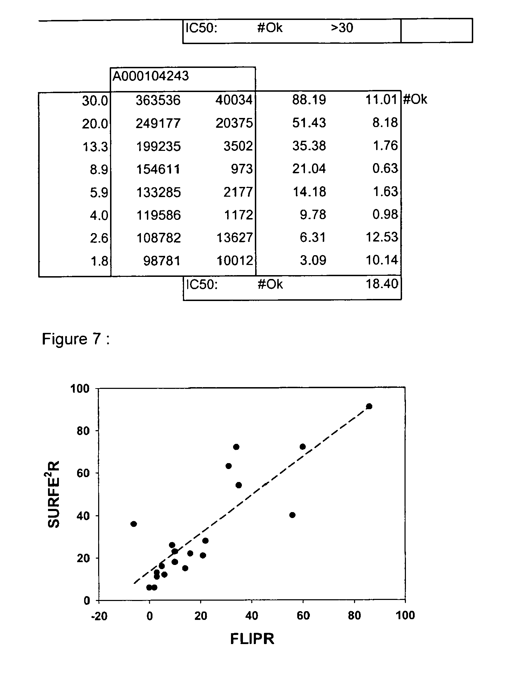

The comparison of the data derived from the fluorescence-based assay with a direct electrophysiology method (Iongate's SURFE$^2$R technology) is the best way to estimate the performance of this assay. The correlation of these two very different techniques is quite good (Figure 7).
The Inhibition measured with the SURFE$^2$R was higher (mean 14 %) except for one compound than the inhibition derived from the indirect FLIPR assay.

DESCRIPTION OF THE FIGURES

Figure 1:

(a) Amino acid sequence of NCX1 represented by SEQ ID NO: 1.
(b) Amino acid sequence of NCX2 represented by SEQ ID NO: 2.
(c) Amino acid sequence of NCX3 represented by SEQ ID NO: 3.

Figure 2:

Fluorescence signal of the CHO-NCX1 cells after Ionomycine addition. Inhibition of NCX1 (high control, 30 $\mu$M A000135933, dashed line) leads to a fluorescence increase due to an increase of cytosolic calcium. Active NCX1 establish the initial calcium load after a few seconds (low control, solid line).

Figure 3:

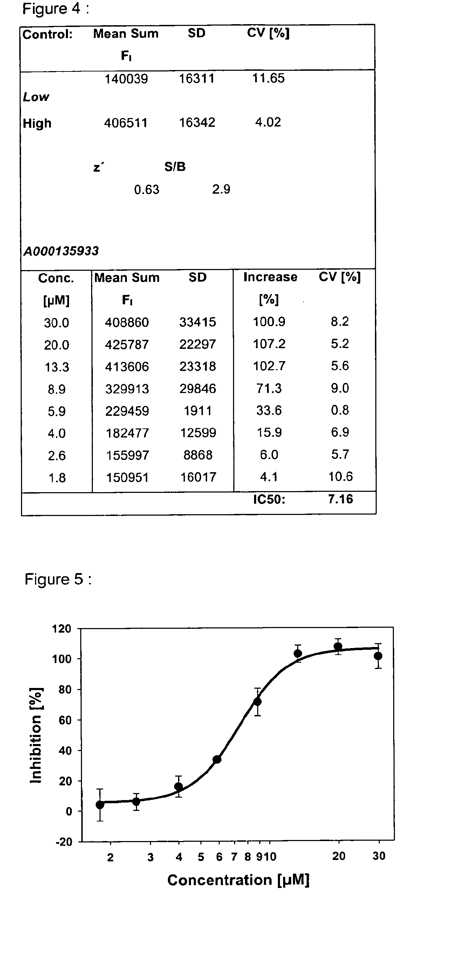

Raw data: Kinetic of the fluorescence changes after ionomycine addition for different concentrations of A000135933. The sum of the fluorescence values from 50 to 90s were used to calculate the percentage fluorescence changes in comparison to the controls. The results are shown in figure 4.

Figure 4:

Assay statistic for a 96 well plate with high and low controls and different concentrations of A000135933. Calculated signal to background ratio (S/B), z' and increase of the fluorescence between 50 and 90 seconds of different concentrations of A000135933 are listed (s.a. Figure 2). For this example the calculated $IC_{50}$ of A000135933 was 7.16 $\mu$M (mean $IC_{50}$: 5.9 $\mu$M).

Figure 5:

Illustration of the percentage fluorescence increase in comparison to the compound concentration of A000135933 and the corresponding fit curve. For this example the calculated $IC_{50}$ of A000135933 was 7.16 $\mu$M (mean $IC_{50}$: 5.9 $\mu$M).

Figure 6:

Raw data print out from the FLIPR.

Figure 7:

Correlation between the NCX1 fluorescence based FLIPR assay with the electrophysiology based SURFE$^2$R technology of one compound class. The inhibition of NCX1 was measured in both cases at 10 $\mu$M.

Sequence Listing

<110> Sanofi-Aventis

<120> Fluorescence based assay to detect sodium/calcium exchanger
"forward mode" modulating compounds

<130> DE2008/025

<140>

<141>

<160> 3


<210> 1
<211> 973
<212> PRT
<213> Homo sapiens

<400> 1
Met Tyr Asn Met Arg Arg Leu Ser Leu Ser Pro Thr Phe Ser Met Gly
Phe His Leu Leu Val Thr Val Ser Leu Leu Phe Ser His Val Asp His
Val Ile Ala Glu Thr Glu Met Glu Gly Glu Gly Asn Glu Thr Gly Glu
Cys Thr Gly Ser Tyr Tyr Cys Lys Lys Gly Val Ile Leu Pro Ile Trp
Glu Pro Gln Asp Pro Ser Phe Gly Asp Lys Ile Ala Arg Ala Thr Val
Tyr Phe Val Ala Met Val Tyr Met Phe Leu Gly Val Ser Ile Ile Ala
Asp Arg Phe Met Ser Ser Ile Glu Val Ile Thr Ser Gln Glu Lys Glu
Ile Thr Ile Lys Lys Pro Asn Gly Glu Thr Thr Lys Thr Thr Val Arg
Ile Trp Asn Glu Thr Val Ser Asn Leu Thr Leu Met Ala Leu Gly Ser
Ser Ala Pro Glu Ile Leu Leu Ser Val Ile Glu Val Cys Gly His Asn
Phe Thr Ala Gly Asp Leu Gly Pro Ser Thr Ile Val Gly Ser Ala Ala
Phe Asn Met Phe Ile Ile Ile Ala Leu Cys Val Tyr Val Val Pro Asp
Gly Glu Thr Arg Lys Ile Lys His Leu Arg Val Phe Phe Val Thr Ala
Ala Trp Ser Ile Phe Ala Tyr Thr Trp Leu Tyr Ile Ile Leu Ser Val
Ile Ser Pro Gly Val Val Glu Val Trp Glu Gly Leu Leu Thr Phe Phe
Phe Phe Pro Ile Cys Val Val Phe Ala Trp Val Ala Asp Arg Arg Leu
Leu Phe Tyr Lys Tyr Val Tyr Lys Arg Tyr Arg Ala Gly Lys Gln Arg
Gly Met Ile Ile Glu His Glu Gly Asp Arg Pro Ser Ser Lys Thr Glu
Ile Glu Met Asp Gly Lys Val Val Asn Ser His Val Glu Asn Phe Leu
Asp Gly Ala Leu Val Leu Glu Val Asp Glu Arg Asp Gln Asp Asp Glu
Glu Ala Arg Arg Glu Met Ala Arg Ile Leu Lys Glu Leu Lys Gln Lys
His Pro Asp Lys Glu Ile Glu Gln Leu Ile Glu Leu Ala Asn Tyr Gln
Val Leu Ser Gln Gln Gln Lys Ser Arg Ala Phe Tyr Arg Ile Gln Ala
Thr Arg Leu Met Thr Gly Ala Gly Asn Ile Leu Lys Arg His Ala Ala
Asp Gln Ala Arg Lys Ala Val Ser Met His Glu Val Asn Thr Glu Val
Thr Glu Asn Asp Pro Val Ser Lys Ile Phe Phe Glu Gln Gly Thr Tyr
Gln Cys Leu Glu Asn Cys Gly Thr Val Ala Leu Thr Ile Ile Arg Arg
Gly Gly Asp Leu Thr Asn Thr Val Phe Val Asp Phe Arg Thr Glu Asp
Gly Thr Ala Asn Ala Gly Ser Asp Tyr Glu Phe Thr Glu Gly Thr Val
Val Phe Lys Pro Gly Asp Thr Gln Lys Glu Ile Arg Val Gly Ile Ile
Asp Asp Asp Ile Phe Glu Glu Asp Glu Asn Phe Leu Val His Leu Ser
Asn Val Lys Val Ser Ser Glu Ala Ser Glu Asp Gly Ile Leu Glu Ala

```
Asn His Val Ser Thr Leu Ala Cys Leu Gly Ser Pro Ser Thr Ala Thr
Val Thr Ile Phe Asp Asp Asp His Ala Gly Ile Phe Thr Phe Glu Glu
Pro Val Thr His Val Ser Glu Ser Ile Gly Ile Met Glu Val Lys Val
Leu Arg Thr Ser Gly Ala Arg Gly Asn Val Ile Val Pro Tyr Lys Thr
Ile Glu Gly Thr Ala Arg Gly Gly Gly Glu Asp Phe Glu Asp Thr Cys
Gly Glu Leu Glu Phe Gln Asn Asp Glu Ile Val Lys Thr Ile Ser Val
Lys Val Ile Asp Asp Glu Glu Tyr Glu Lys Asn Lys Thr Phe Phe Leu
Glu Ile Gly Glu Pro Arg Leu Val Glu Met Ser Glu Lys Lys Ala Leu
Leu Leu Asn Glu Leu Gly Gly Phe Thr Ile Thr Gly Lys Tyr Leu Phe
Gly Gln Pro Val Phe Arg Lys Val His Ala Arg Glu His Pro Ile Leu
Ser Thr Val Ile Thr Ile Ala Asp Glu Tyr Asp Asp Lys Gln Pro Leu
Thr Ser Lys Glu Glu Glu Glu Arg Arg Ile Ala Glu Met Gly Arg Pro
Ile Leu Gly Glu His Thr Lys Leu Glu Val Ile Ile Glu Glu Ser Tyr
Glu Phe Lys Ser Thr Val Asp Lys Leu Ile Lys Lys Thr Asn Leu Ala
Leu Val Val Gly Thr Asn Ser Trp Arg Glu Gln Phe Ile Glu Ala Ile
Thr Val Ser Ala Gly Glu Asp Asp Asp Asp Glu Cys Gly Glu Glu
Lys Leu Pro Ser Cys Phe Asp Tyr Val Met His Phe Leu Thr Val Phe
Trp Lys Val Leu Phe Ala Phe Val Pro Pro Thr Glu Tyr Trp Asn Gly
Trp Ala Cys Phe Ile Val Ser Ile Leu Met Ile Gly Leu Leu Thr Ala
Phe Ile Gly Asp Leu Ala Ser His Phe Gly Cys Thr Ile Gly Leu Lys
Asp Ser Val Thr Ala Val Val Phe Val Ala Leu Gly Thr Ser Val Pro
Asp Thr Phe Ala Ser Lys Val Ala Ala Thr Gln Asp Gln Tyr Ala Asp
Ala Ser Ile Gly Asn Val Thr Gly Ser Asn Ala Val Asn Val Phe Leu
Gly Ile Gly Val Ala Trp Ser Ile Ala Ala Ile Tyr His Ala Ala Asn
Gly Glu Gln Phe Lys Val Ser Pro Gly Thr Leu Ala Phe Ser Val Thr
Leu Phe Thr Ile Phe Ala Phe Ile Asn Val Gly Val Leu Leu Tyr Arg
Arg Arg Pro Glu Ile Gly Gly Glu Leu Gly Gly Pro Arg Thr Ala Lys
Leu Leu Thr Ser Cys Leu Phe Val Leu Leu Trp Leu Leu Tyr Ile Phe
Phe Ser Ser Leu Glu Ala Tyr Cys His Ile Lys Gly Phe
```

<210> 2
<211> 921
<212> PRT
<213> Homo sapiens

<400> 2
```
Met Ala Pro Leu Ala Leu Val Gly Val Thr Leu Leu Leu Ala Ala Pro
Pro Cys Ser Gly Ala Ala Thr Pro Thr Pro Ser Leu Pro Pro Pro Pro
Ala Asn Asp Ser Asp Thr Ser Thr Gly Gly Cys Gln Gly Ser Tyr Arg
Cys Gln Pro Gly Val Leu Leu Pro Val Trp Glu Pro Asp Asp Pro Ser
Leu Gly Asp Lys Ala Ala Arg Ala Val Val Tyr Phe Val Ala Met Val
Tyr Met Phe Leu Gly Val Ser Ile Ile Ala Asp Arg Phe Met Ala Ala
Ile Glu Val Ile Thr Ser Lys Glu Lys Glu Ile Thr Ile Thr Lys Ala
Asn Gly Glu Thr Ser Val Gly Thr Val Arg Ile Trp Asn Glu Thr Val
Ser Asn Leu Thr Leu Met Ala Leu Gly Ser Ser Ala Pro Glu Ile Leu
Leu Ser Val Ile Glu Val Cys Gly His Asn Phe Gln Ala Gly Glu Leu
Gly Pro Gly Thr Ile Val Gly Ser Ala Ala Phe Asn Met Phe Val Val
Ile Ala Val Cys Ile Tyr Val Ile Pro Ala Gly Glu Ser Arg Lys Ile
Lys His Leu Arg Val Phe Phe Val Thr Ala Ser Trp Ser Ile Phe Ala
Tyr Val Trp Leu Tyr Leu Ile Leu Ala Val Phe Ser Pro Gly Val Val
Gln Val Trp Glu Ala Leu Leu Thr Leu Val Phe Phe Pro Val Cys Val
Val Phe Ala Trp Met Ala Asp Lys Arg Leu Leu Phe Tyr Lys Tyr Val
```

```
Tyr Lys Arg Tyr Arg Thr Asp Pro Arg Ser Gly Ile Ile Ile Gly Ala
Glu Gly Asp Pro Pro Lys Ser Ile Glu Leu Asp Gly Thr Phe Val Gly
Ala Glu Ala Pro Gly Glu Leu Gly Gly Leu Gly Pro Gly Pro Ala Glu
Ala Arg Glu Leu Asp Ala Ser Arg Arg Glu Val Ile Gln Ile Leu Lys
Asp Leu Lys Gln Lys His Pro Asp Lys Asp Leu Glu Gln Leu Val Gly
Ile Ala Asn Tyr Tyr Ala Leu Leu His Gln Gln Lys Ser Arg Ala Phe
Tyr Arg Ile Gln Ala Thr Arg Leu Met Thr Gly Ala Gly Asn Val Leu
Arg Arg His Ala Ala Asp Ala Ser Arg Arg Ala Ala Pro Ala Glu Gly
Ala Gly Glu Asp Glu Asp Asp Gly Ala Ser Arg Ile Phe Phe Glu Pro
Ser Leu Tyr His Cys Leu Glu Asn Cys Gly Ser Val Leu Leu Ser Val
Thr Cys Gln Gly Gly Glu Gly Asn Ser Thr Phe Tyr Val Asp Tyr Arg
Thr Glu Asp Gly Ser Ala Lys Ala Gly Ser Asp Tyr Glu Tyr Ser Glu
Gly Thr Leu Val Phe Lys Pro Gly Glu Thr Gln Lys Glu Leu Arg Ile
Gly Ile Ile Asp Asp Asp Ile Phe Glu Glu Asp Glu His Phe Phe Val
Arg Leu Leu Asn Leu Arg Val Gly Asp Ala Gln Gly Met Phe Glu Pro
Asp Gly Gly Gly Arg Pro Lys Gly Arg Leu Val Ala Pro Leu Leu Ala
Thr Val Thr Ile Leu Asp Asp Asp His Ala Gly Ile Phe Ser Phe Gln
Asp Arg Leu Leu His Val Ser Glu Cys Met Gly Thr Val Asp Val Arg
Val Val Arg Ser Ser Gly Ala Arg Gly Thr Val Arg Leu Pro Tyr Arg
Thr Val Asp Gly Thr Ala Arg Gly Gly Gly Val His Tyr Glu Asp Ala
Cys Gly Glu Leu Glu Phe Gly Asp Asp Glu Thr Met Lys Thr Leu Gln
Val Lys Ile Val Asp Asp Glu Glu Tyr Glu Lys Lys Asp Asn Phe Phe
Ile Glu Leu Gly Gln Pro Gln Trp Leu Lys Arg Gly Ile Ser Ala Leu
Leu Leu Asn Gln Gly Asp Gly Asp Arg Lys Leu Thr Ala Glu Glu Glu
Glu Ala Arg Arg Ile Ala Glu Met Gly Lys Pro Val Leu Gly Glu Asn
Cys Arg Leu Glu Val Ile Ile Glu Glu Ser Tyr Asp Phe Lys Asn Thr
Val Asp Lys Leu Ile Lys Lys Thr Asn Leu Ala Leu Val Ile Gly Thr
His Ser Trp Arg Glu Gln Phe Leu Glu Ala Ile Thr Val Ser Ala Gly
Asp Glu Glu Glu Glu Glu Asp Gly Ser Arg Glu Glu Arg Leu Pro Ser
Cys Phe Asp Tyr Val Met His Phe Leu Thr Val Phe Trp Lys Val Leu
Phe Ala Cys Val Pro Pro Thr Glu Tyr Cys His Gly Trp Ala Cys Phe
Gly Val Ser Ile Leu Val Ile Gly Leu Leu Thr Ala Leu Ile Gly Asp
Leu Ala Ser His Phe Gly Cys Thr Val Gly Leu Lys Asp Ser Val Asn
Ala Val Val Phe Val Ala Leu Gly Thr Ser Ile Pro Asp Thr Phe Ala
Ser Lys Val Ala Ala Leu Gln Asp Gln Cys Ala Asp Ala Ser Ile Gly
Asn Val Thr Gly Ser Asn Ala Val Asn Val Phe Leu Gly Leu Gly Val
Ala Trp Ser Val Ala Ala Val Tyr Trp Ala Val Gln Gly Arg Pro Phe
Glu Val Arg Thr Gly Thr Leu Ala Phe Ser Val Thr Leu Phe Thr Val
Phe Ala Phe Val Gly Ile Ala Val Leu Leu Tyr Arg Arg Arg Pro His
Ile Gly Gly Glu Leu Gly Gly Pro Arg Gly Pro Lys Leu Ala Thr Thr
Ala Leu Phe Leu Gly Leu Trp Leu Leu Tyr Ile Leu Phe Ala Ser Leu
Glu Ala Tyr Cys His Ile Arg Gly
Phe
```

```
<210> 3
<211> 924
<212> PRT
<213> Homo sapiens

<400> 3
Met Ala Trp Leu Arg Leu Gln Pro Leu Thr Ser Ala Phe Leu His Phe
Gly Leu Val Thr Phe Val Leu Phe Leu Asn Gly Leu Arg Ala Glu Ala
Gly Gly Ser Gly Asp Val Pro Ser Thr Gly Gln Asn Asn Glu Ser Cys
```

```
Ser Gly Ser Ser Asp Cys Lys Glu Gly Val Ile Leu Pro Ile Trp Tyr
Pro Glu Asn Pro Ser Leu Gly Asp Lys Ile Ala Arg Val Ile Val Tyr
Phe Val Ala Leu Ile Tyr Met Phe Leu Gly Val Ser Ile Ile Ala Asp
Arg Phe Met Ala Ser Ile Glu Val Ile Thr Ser Gln Glu Arg Glu Val
Thr Ile Lys Lys Pro Asn Gly Glu Thr Ser Thr Thr Thr Ile Arg Val
Trp Asn Glu Thr Val Ser Asn Leu Thr Leu Met Ala Leu Gly Ser Ser
Ala Pro Glu Ile Leu Leu Ser Leu Ile Glu Val Cys Gly His Gly Phe
Ile Ala Gly Asp Leu Gly Pro Ser Thr Ile Val Gly Ser Ala Ala Phe
Asn Met Phe Ile Ile Ile Gly Ile Cys Val Tyr Val Ile Pro Asp Gly
Glu Thr Arg Lys Ile Lys His Leu Arg Val Phe Phe Ile Thr Ala Ala
Trp Ser Ile Phe Ala Tyr Ile Trp Leu Tyr Met Ile Leu Ala Val Phe
Ser Pro Gly Val Val Gln Val Trp Glu Gly Leu Leu Thr Leu Phe Phe
Phe Pro Val Cys Val Leu Leu Ala Trp Val Ala Asp Lys Arg Leu Leu
Phe Tyr Lys Tyr Met His Lys Lys Tyr Arg Thr Asp Lys His Arg Gly
Ile Ile Ile Glu Thr Glu Gly Asp His Pro Lys Gly Ile Glu Met Asp
Gly Lys Met Met Asn Ser His Phe Leu Asp Gly Asn Leu Val Pro Leu
Glu Gly Lys Glu Val Asp Glu Ser Arg Arg Glu Met Ile Arg Ile Leu
Lys Asp Leu Lys Gln Lys His Pro Glu Lys Asp Leu Asp Gln Leu Val
Glu Met Ala Asn Tyr Tyr Ala Leu Ser His Gln Gln Lys Ser Arg Ala
Phe Tyr Arg Ile Gln Ala Thr Arg Met Met Thr Gly Ala Gly Asn Ile
Leu Lys Lys His Ala Ala Glu Gln Ala Lys Lys Ala Ser Ser Met Ser
Glu Val His Thr Asp Glu Pro Glu Asp Phe Ile Ser Lys Val Phe Phe
Asp Pro Cys Ser Tyr Gln Cys Leu Glu Asn Cys Gly Ala Val Leu Leu
Thr Val Val Arg Lys Gly Gly Asp Met Ser Lys Thr Met Tyr Val Asp
Tyr Lys Thr Glu Asp Gly Ser Ala Asn Ala Gly Ala Asp Tyr Glu Phe
Thr Glu Gly Thr Val Val Leu Lys Pro Gly Glu Thr Gln Lys Glu Phe
Ser Val Gly Ile Ile Asp Asp Asp Ile Phe Glu Glu Asp Glu His Phe
Phe Val Arg Leu Ser Asn Val Arg Ile Glu Glu Glu Gln Pro Glu Glu
Gly Met Pro Pro Ala Ile Phe Asn Ser Leu Pro Leu Pro Arg Ala Val
Leu Ala Ser Pro Cys Val Ala Thr Val Thr Ile Leu Asp Asp Asp His
Ala Gly Ile Phe Thr Phe Glu Cys Asp Thr Ile His Val Ser Glu Ser
Ile Gly Val Met Glu Val Lys Val Leu Arg Thr Ser Gly Ala Arg Gly
Thr Val Ile Val Pro Phe Arg Thr Val Glu Gly Thr Ala Lys Gly Gly
Gly Glu Asp Phe Glu Asp Thr Tyr Gly Glu Leu Glu Phe Lys Asn Asp
Glu Thr Val Lys Thr Ile Arg Val Lys Ile Val Asp Glu Glu Glu Tyr
Glu Arg Gln Glu Asn Phe Phe Ile Ala Leu Gly Glu Pro Lys Trp Met
Glu Arg Gly Ile Ser Ala Leu Leu Leu Ser Pro Asp Arg Lys Leu Thr
Met Glu Glu Glu Glu Ala Lys Arg Ile Ala Glu Met Gly Lys Pro Val
Leu Gly Glu His Pro Lys Leu Glu Val Ile Ile Glu Glu Ser Tyr Glu
Phe Lys Thr Thr Val Asp Lys Leu Ile Lys Lys Thr Asn Leu Ala Leu
Val Val Gly Thr His Ser Trp Arg Asp Gln Phe Met Glu Ala Ile Thr
Val Ser Ala Ala Gly Asp Glu Asp Glu Asp Glu Ser Gly Glu Glu Arg
Leu Pro Ser Cys Phe Asp Tyr Val Met His Phe Leu Thr Val Phe Trp
Lys Val Leu Phe Ala Cys Val Pro Pro Thr Glu Tyr Cys His Gly Trp
Ala Cys Phe Ala Val Ser Ile Leu Ile Ile Gly Met Leu Thr Ala Ile
Ile Gly Asp Leu Ala Ser His Phe Gly Cys Thr Ile Gly Leu Lys Asp
Ser Val Thr Ala Val Val Phe Val Ala Phe Gly Thr Ser Val Pro Asp
Thr Phe Ala Ser Lys Ala Ala Ala Leu Gln Asp Val Tyr Ala Asp Ala
Ser Ile Gly Asn Val Thr Gly Ser Asn Ala Val Asn Val Phe Leu Gly
Ile Gly Leu Ala Trp Ser Val Ala Ala Ile Tyr Trp Ala Leu Gln Gly
Gln Glu Phe His Val Ser Ala Gly Thr Leu Ala Phe Ser Val Thr Leu
Phe Thr Ile Phe Ala Phe Val Cys Ile Ser Val Leu Leu Tyr Arg Arg
Arg Pro His Leu Gly Gly Glu Leu Gly Gly Pro Arg Gly Cys Lys Leu
Ala Thr Thr Trp Leu Phe Val Ser Leu Trp Leu Leu Tyr Ile Leu Phe
```

**Ala Thr Leu Glu Ala Tyr Cys Tyr Ile Lys Gly Phe**


Application Project
--------------------
<110> Sanofi-Aventis

<120> Title : Fluorescence based assay to detect sodium-calcium exchanger (NCX)
            "forward mode" modulating compounds
<130> AppFileReference : DE2008/025

<140> CurrentAppNumber :

<141> CurrentFilingDate :

<160> 3

Sequence
--------
<213> OrganismName : Homo sapiens
<400> PreSequenceString :

| | | | | | | |
|---|---|---|---|---|---|---|
| MYNMRRLSLS | PTFSMGFHLL | VTVSLLFSHV | DHVIAETEME | GEGNETGECT | GSYYCKKGVI | 60 |
| LPIWEPQDPS | FGDKIARATV | YFVAMVYMFL | GVSIIADRFM | SSIEVITSQE | KEITIKKPNG | 120 |
| ETTKTTVRIW | NETVSNLTLM | ALGSSAPEIL | LSVIEVCGHN | FTAGDLGPST | IVGSAAFNMF | 180 |
| IIIALCVYVV | PDGETRKIKH | LRVFFVTAAW | SIFAYTWLYI | ILSVISPGVV | EVWEGLLTFF | 240 |
| FFPICVVFAW | VADRRLLFYK | YVYKRYRAGK | QRGMIIEHEG | DRPSSKTEIE | MDGKVVNSHV | 300 |
| ENFLDGALVL | EVDERDQDDE | EARREMARIL | KELKQKHPDK | EIEQLIELAN | YQVLSQQQKS | 360 |
| RAFYRIQATR | LMTGAGNILK | RHAADQARKA | VSMHEVNTEV | TENDPVSKIF | FEQGTYQCLE | 420 |
| NCGTVALTII | RRGGDLTNTV | FVDFRTEDGT | ANAGSDYEFT | EGTVVFKPGD | TQKEIRVGII | 480 |
| DDDIFEEDEN | FLVHLSNVKV | SSEASEDGIL | EANHVSTLAC | LGSPSTATVT | IFDDDHAGIF | 540 |
| TFEEPVTHVS | ESIGIMEVKV | LRTSGARGNV | IVPYKTIEGT | ARGGGEDFED | TCGELEFQND | 600 |
| EIVKTISVKV | IDDEEYEKNK | TFFLEIGEPR | LVEMSEKKAL | LLNELGGFTI | TGKYLFGQPV | 660 |
| FRKVHAREHP | ILSTVITIAD | EYDDKQPLTS | KEEEERRIAE | MGRPILGEHT | KLEVIIEESY | 720 |
| EFKSTVDKLI | KKTNLALVVG | TNSWREQFIE | AITVSAGEDD | DDDECGEEKL | PSCFDYVMHF | 780 |
| LTVFWKVLFA | FVPPTEYWNG | WACFIVSILM | IGLLTAFIGD | LASHFGCTIG | LKDSVTAVVF | 840 |
| VALGTSVPDT | FASKVAATQD | QYADASIGNV | TGSNAVNVFL | GIGVAWSIAA | IYHAANGEQF | 900 |
| KVSPGTLAFS | VTLFTIFAFI | NVGVLLYRRR | PEIGGELGGP | RTAKLLTSCL | FVLLWLLYIF | 960 |
| FSSLEAYCHI | KGF | | | | | 973 |

<212> Type : PRT
<211> Length : 973
        SequenceName : SEQ ID NO 1
        SequenceDescription :


Sequence
--------
<213> OrganismName : Homo sapiens
<400> PreSequenceString :

| | | | | | | |
|---|---|---|---|---|---|---|
| MAPLALVGVT | LLLAAPPCSG | AATPTPSLPP | PPANDSDTST | GGCQGSYRCQ | PGVLLPVWEP | 60 |
| DDPSLGDKAA | RAVVYFVAMV | YMFLGVSIIA | DRFMAAIEVI | TSKEKEITIT | KANGETSVGT | 120 |
| VRIWNETVSN | LTLMALGSSA | PEILLSVIEV | CGHNFQAGEL | GPGTIVGSAA | FNMFVVIAVC | 180 |
| IYVIPAGESR | KIKHLRVFFV | TASWSIFAYV | WLYLILAVFS | PGVVQVWEAL | LTLVFFPVCV | 240 |
| VFAWMADKRL | LFYKYVYKRY | RTDPRSGIII | GAEGDPPKSI | ELDGTFVGAE | APGELGGLGP | 300 |
| GPAEARELDA | SRREVIQILK | DLKQKHPDKD | LEQLVGIANY | YALLHQQKSR | AFYRIQATRL | 360 |
| MTGAGNVLRR | HAADASRRAA | PAEGAGEDED | DGASRIFFEP | SLYHCLENCG | SVLLSVTCQG | 420 |
| GEGNSTFYVD | YRTEDGSAKA | GSDYEYSEGT | LVFKPGETQK | ELRIGIIDDD | IFEEDEHFFV | 480 |
| RLLNLRVGDA | QGMFEPDGGG | RPKGRLVAPL | LATVTILDDD | HAGIFSFQDR | LLHVSECMGT | 540 |
| VDVRVVRSSG | ARGTVRLPYR | TVDGTARGGG | VHYEDACGEL | EFGDDETMKT | LQVKIVDDEE | 600 |
| YEKKDNFFIE | LGQPQWLKRG | ISALLLNQGD | GDRKLTAEEE | EARRIAEMGK | PVLGENCRLE | 660 |
| VIIEESYDFK | NTVDKLIKKT | NLALVIGTHS | WREQFLEAIT | VSAGDEEEEE | DGSREERLPS | 720 |
| CFDYVMHFLT | VFWKVLFACV | PPTEYCHGWA | CFGVSILVIG | LLTALIGDLA | SHFGCTVGLK | 780 |
| DSVNAVVFVA | LGTSIPDTFA | SKVAALQDQC | ADASIGNVTG | SNAVNVFLGL | GVAWSAAVY | 840 |
| WAVQGRPFEV | RTGTLAFSVT | LFTVFAFVGI | AVLLYRRRPH | IGGELGGPRG | PKLATTALFL | 900 |
| GLWLLYILFA | SLEAYCHIRG | F | | | | 921 |

<212> Type : PRT
<211> Length : 921
        SequenceName : SEQ ID NO 2
        SequenceDescription :


Sequence
--------
<213> OrganismName : Homo sapiens

```
<400> PreSequenceString :
MAWLRLQPLT SAFLHFGLVT FVLFLNGLRA EAGGSGDVPS TGQNNESCSG SSDCKEGVIL      60
PIWYPENPSL GDKIARVIVY FVALIYMFLG VSIIADRFMA SIEVITSQER EVTIKKPNGE     120
TSTTTIRVWN ETVSNLTLMA LGSSAPEILL SLIEVCGHGF IAGDLGPSTI VGSAAFNMFI     180
IIGICVYVIP DGETRKIKHL RVFFITAAWS IFAYIWLYMI LAVFSPGVVQ VWEGLLTLFF     240
FPVCVLLAWV ADKRLLFYKY MHKKYRTDKH RGIIIETEGD HPKGIEMDGK MMNSHFLDGN     300
LVPLEGKEVD ESRREMIRIL KDLKQKHPEK DLDQLVEMAN YYALSHQQKS RAFYRIQATR     360
MMTGAGNILK KHAAEQAKKA SSMSEVHTDE PEDFISKVFF DPCSYQCLEN CGAVLLTVVR     420
KGGDMSKTMY VDYKTEDGSA NAGADYEFTE GTVVLKPGET QKEFSVGIID DDIFEEDEHF     480
FVRLSNVRIE EEQPEEGMPP AIFNSLPLPR AVLASPCVAT VTILDDDHAG IFTFECDTIH     540
VSESIGVMEV KVLRTSGARG TVIVPFRTVE GTAKGGGEDF EDTYGELEFK NDETVKTIRV     600
KIVDEEEYER QENFFIALGE PKWMERGISA LLLSPDRKLT MEEEEAKRIA EMGKPVLGEH     660
PKLEVIIEES YEFKTTVDKL IKKTNLALVV GTHSWRDQFM EAITVSAAGD EDEDESGEER     720
LPSCFDYVMH FLTVFWKVLF ACVPPTEYCH GWACFAVSIL IIGMLTAIIG DLASHFGCTI     780
GLKDSVTAVV FVAFGTSVPD TFASKAAALQ DVYADASIGN VTGSNAVNVF LGIGLAWSVA     840
AIYWALQGQE FHVSAGTLAF SVTLFTIFAF VCISVLLYRR RPHLGGELGG PRGCKLATTW     900
LFVSLWLLYI LFATLEAYCY IKGF                                            924
<212> Type : PRT
<211> Length : 924
      SequenceName : SEQ ID NO 3
      SequenceDescription :
```

**Claims**

1. An assay for determining the activity of a sodium/calcium exchanger comprising:

    e) providing cells expressing a sodium/calcium exchanger;
    f) providing a colored substance for determining intracellular calcium;
    g) contacting cells with a sodium/calcium exchanger activator; and
    h) comparing the calcium mediated change in the luminescent signal from said colored substance to a luminescent signal produced in a control experiment.

2. The assay according to claim 1, wherein the sodium/calcium exchanger is a NCX protein of the following list: NCX1, NCX2, NCX3; or a NCKX protein of the following list: NCKX1, NCKX2, NCKX3, NCKX4, NCKX5.

3. The assay according to claim 1, wherein the sodium/calcium exchanger is a NCX protein of the following list: NCX1, NCX2, NCX3.

4. The assay according to claims 1 to 3, wherein the sodium/calcium exchanger is of mammalian origin, preferably from rat, mouse, dog, bovine, pig, ape or human.

5. The assay according to claims 1 to 4, wherein the cells are selected from the group consisting of: CHO, HEK, COS7 and JURKAT cells.

6. The assay according to claims 1 to 5, wherein said colored substance is added to the cells as a dye precursor capable of entering the cells and being hydrolyzed to a dye, whereby the dye complexes with calcium in said cells and provides a luminescent signal.

7. The assay according to claims 1 to 6, wherein said luminescent signal is fluorescence and said monitoring step c) employs a FLIPR device.

8. The assay according to claim 6, wherein said dye precursor is an acetoxymethylester derivate.

9. The assay according to claim 6, wherein said dye is the calcium sensitive fluorescence dye fluo-4.

10. The assay according to claims 1 to 9, wherein said sodium/calcium exchanger activator is ionomycin.

**11.** Use of the assay according to claims 1 to 10 to test a compound for activity as an agonist or antagonist of a sodium/calcium exchanger.

**12.** Use of the assay according to claims 1 to 10 for the diagnosis of a disease associated with a sodium/calcium exchanger altered expression.

**13.** An assay for determining the activity of a sodium/calcium exchanger in response to the addition of a compound comprising:

a) providing cells expressing a sodium/calcium exchanger;
b) providing a colored substance for determining intracellular calcium;
c) contacting cells with a compound, wherein said cells have been treated, prior to treating with said compound, with a sodium/calcium exchanger activator; and
d) comparing the calcium mediated change in the luminescent signal from said colored substance to a luminescent signal produced in a control experiment.

**14.** The assay according to claim 13, wherein the sodium/calcium exchanger is a NCX protein of the following list: NCX1, NCX2, NCX3; or a NCKX protein of the following list: NCKX1, NCKX2, NCKX3, NCKX4, NCKX5.

**15.** The assay according to claim 13, wherein the sodium/calcium exchanger is a NCX protein of the following list: NCX1, NCX2, NCX3.

**16.** The assay according to claims 13 and 15, wherein the sodium/calcium exchanger is of mammalian origin, preferably from rat, mouse, dog, bovine, pig, ape or human.

**17.** The assay according to claims 13 to 16, wherein the cells are selected from the group consisting of: CHO, HEK, COS7 and JURKAT cells.

**18.** The assay according to claims 13 to 17, wherein said colored substance is added to the cells as a dye precursor capable of entering the cells and being hydrolyzed to a dye, whereby the dye complexes with calcium in said cells and provides a luminescent signal.

**20.** The assay according to claim 18, wherein said dye precursor is an acetoxymethylester derivate.

**21.** The assay according to claim 18, wherein said dye is the calcium sensitive fluorescence dye fluo-4.

**22.** The assay according to claims 13 to 21, wherein said compound is a sodium/calcium exchanger antagonist.

**23.** The assay according to claims 13 to 22, wherein said sodium/calcium exchanger activator is ionomycin.

**24.** A kit of parts comprising:

a) lyophilized cells expriming a sodium/calcium exchanger;
b) a colored substance;
c) a compound buffer; and
d) a colored substance buffer.

**25.** The kit of parts according to claim 24, wherein said colored substance is the calcium sensitive fluorescence dye fluo-4.

**26.** The kit of parts according to claims 24 and 25, wherein the sodium/calcium exchanger is a NCX protein of the following list: NCX1, NCX2, NCX3; or a NCKX protein of the following list: NCKX1, NCKX2, NCKX3, NCKX4, NCKX5.

**27.** The kit of parts according to claims 24 to 26, wherein the sodium/calcium exchanger is of mammalian origin, preferably from rat, mouse, dog, bovine, pig, ape or human.

**28.** Use of a kit of parts according to claims 24 to 27 to test a compound for activity as an agonist or antagonist of a sodium/calcium exchanger.

**29.** Use of a kit of parts according to claims 24 to 27 for the diagnosis of a disease associated with a sodium/calcium exchanger altered expression.

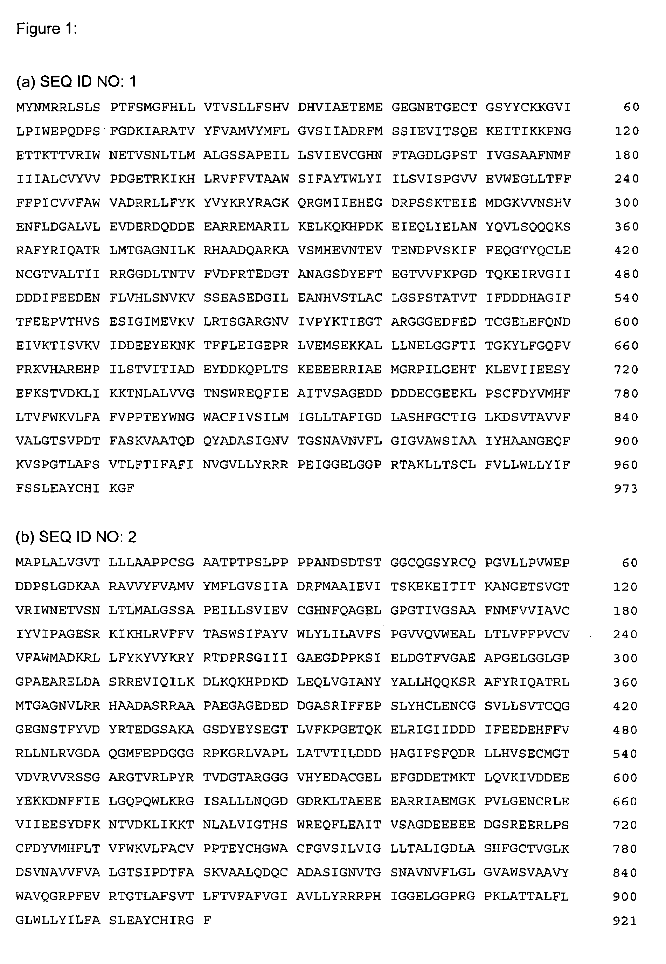

Figure 1:

(a) SEQ ID NO: 1

```
MYNMRRLSLS PTFSMGFHLL VTVSLLFSHV DHVIAETEME GEGNETGECT GSYYCKKGVI      60
LPIWEPQDPS FGDKIARATV YFVAMVYMFL GVSIIADRFM SSIEVITSQE KEITIKKPNG     120
ETTKTTVRIW NETVSNLTLM ALGSSAPEIL LSVIEVCGHN FTAGDLGPST IVGSAAFNMF     180
IIIALCVYVV PDGETRKIKH LRVFFVTAAW SIFAYTWLYI ILSVISPGVV EVWEGLLTFF     240
FFPICVVFAW VADRRLLFYK YVYKRYRAGK QRGMIIEHEG DRPSSKTEIE MDGKVVNSHV     300
ENFLDGALVL EVDERDQDDE EARREMARIL KELKQKHPDK EIEQLIELAN YQVLSQQQKS     360
RAFYRIQATR LMTGAGNILK RHAADQARKA VSMHEVNTEV TENDPVSKIF FEQGTYQCLE     420
NCGTVALTII RRGGDLTNTV FVDFRTEDGT ANAGSDYEFT EGTVVFKPGD TQKEIRVGII     480
DDDIFEEDEN FLVHLSNVKV SSEASEDGIL EANHVSTLAC LGSPSTATVT IFDDDHAGIF     540
TFEEPVTHVS ESIGIMEVKV LRTSGARGNV IVPYKTIEGT ARGGGEDFED TCGELEFQND     600
EIVKTISVKV IDDEEYEKNK TFFLEIGEPR LVEMSEKKAL LLNELGGFTI TGKYLFGQPV     660
FRKVHAREHP ILSTVITIAD EYDDKQPLTS KEEEERRIAE MGRPILGEHT KLEVIIEESY     720
EFKSTVDKLI KKTNLALVVG TNSWREQFIE AITVSAGEDD DDDECGEEKL PSCFDYVMHF     780
LTVFWKVLFA FVPPTEYWNG WACFIVSILM IGLLTAFIGD LASHFGCTIG LKDSVTAVVF     840
VALGTSVPDT FASKVAATQD QYADASIGNV TGSNAVNVFL GIGVAWSIAA IYHAANGEQF     900
KVSPGTLAFS VTLFTIFAFI NVGVLLYRRR PEIGGELGGP RTAKLLTSCL FVLLWLLYIF     960
FSSLEAYCHI KGF                                                       973
```

(b) SEQ ID NO: 2

```
MAPLALVGVT LLLAAPPCSG AATPTPSLPP PPANDSDTST GGCQGSYRCQ PGVLLPVWEP      60
DDPSLGDKAA RAVVYFVAMV YMFLGVSIIA DRFMAAIEVI TSKEKEITIT KANGETSVGT     120
VRIWNETVSN LTLMALGSSA PEILLSVIEV CGHNFQAGEL GPGTIVGSAA FNMFVVIAVC     180
IYVIPAGESR KIKHLRVFFV TASWSIFAYV WLYLILAVFS PGVVQVWEAL LTLVFFPVCV     240
VFAWMADKRL LFYKYVYKRY RTDPRSGIII GAEGDPPKSI ELDGTFVGAE APGELGGLGP     300
GPAEARELDA SRREVIQILK DLKQKHPDKD LEQLVGIANY YALLHQQKSR AFYRIQATRL     360
MTGAGNVLRR HAADASRRAA PAEGAGEDED DGASRIFFEP SLYHCLENCG SVLLSVTCQG     420
GEGNSTFYVD YRTEDGSAKA GSDYEYSEGT LVFKPGETQK ELRIGIIDDD IFEEDEHFFV     480
RLLNLRVGDA QGMFEPDGGG RPKGRLVAPL LATVTILDDD HAGIFSFQDR LLHVSECMGT     540
VDVRVVRSSG ARGTVRLPYR TVDGTARGGG VHYEDACGEL EFGDDETMKT LQVKIVDDEE     600
YEKKDNFFIE LGQPQWLKRG ISALLLNQGD GDRKLTAEEE EARRIAEMGK PVLGENCRLE     660
VIIEESYDFK NTVDKLIKKT NLALVIGTHS WREQFLEAIT VSAGDEEEEE DGSREERLPS     720
CFDYVMHFLT VFWKVLFACV PPTEYCHGWA CFGVSILVIG LLTALIGDLA SHFGCTVGLK     780
DSVNAVVFVA LGTSIPDTFA SKVAALQDQC ADASIGNVTG SNAVNVFLGL GVAWSVAAVY     840
WAVQGRPFEV RTGTLAFSVT LFTVFAFVGI AVLLYRRRPH IGGELGGPRG PKLATTALFL     900
GLWLLYILFA SLEAYCHIRG F                                               921
```

(c) SEQ ID NO: 3

```
MAWLRLQPLT  SAFLHFGLVT  FVLFLNGLRA  EAGGSGDVPS  TGQNNESCSG  SSDCKEGVIL    60
PIWYPENPSL  GDKIARVIVY  FVALIYMFLG  VSIIADRFMA  SIEVITSQER  EVTIKKPNGE   120
TSTTTIRVWN  ETVSNLTLMA  LGSSAPEILL  SLIEVCGHGF  IAGDLGPSTI  VGSAAFNMFI   180
IIGICVYVIP  DGETRKIKHL  RVFFITAAWS  IFAYIWLYMI  LAVFSPGVVQ  VWEGLLTLFF   240
FPVCVLLAWV  ADKRLLFYKY  MHKKYRTDKH  RGIIIETEGD  HPKGIEMDGK  MMNSHFLDGN   300
LVPLEGKEVD  ESRREMIRIL  KDLKQKHPEK  DLDQLVEMAN  YYALSHQQKS  RAFYRIQATR   360
MMTGAGNILK  KHAAEQAKKA  SSMSEVHTDE  PEDFISKVFF  DPCSYQCLEN  CGAVLLTVVR   420
KGGDMSKTMY  VDYKTEDGSA  NAGADYEFTE  GTVVLKPGET  QKEFSVGIID  DDIFEEDEHF   480
FVRLSNVRIE  EEQPEEGMPP  AIFNSLPLPR  AVLASPCVAT  VTILDDDHAG  IFTFECDTIH   540
VSESIGVMEV  KVLRTSGARG  TVIVPFRTVE  GTAKGGGEDF  EDTYGELEFK  NDETVKTIRV   600
KIVDEEEYER  QENFFIALGE  PKWMERGISA  LLLSPDRKLT  MEEEEAKRIA  EMGKPVLGEH   660
PKLEVIIEES  YEFKTTVDKL  IKKTNLALVV  GTHSWRDQFM  EAITVSAAGD  EDEDESGEER   720
LPSCFDYVMH  FLTVFWKVLF  ACVPPTEYCH  GWACFAVSIL  IIGMLTAIIG  DLASHFGCTI   780
GLKDSVTAVV  FVAFGTSVPD  TFASKAAALQ  DVYADASIGN  VTGSNAVNVF  LGIGLAWSVA   840
AIYWALQGQE  FHVSAGTLAF  SVTLFTIFAF  VCISVLLYRR  RPHLGGELGG  PRGCKLATTW   900
LFVSLWLLYI  LFATLEAYCY  IKGF                                             924
```

Figure 2 :

Figure 3 :

Figure 4 :

| Control: | Mean Sum $F_l$ | SD | CV [%] | | |
|---|---|---|---|---|---|
| Low | 140039 | 16311 | 11.65 | | |
| High | 406511 | 16342 | 4.02 | | |
| | z′ | | S/B | | |
| | 0.63 | | 2.9 | | |

A000135933

| Conc. [µM] | Mean Sum $F_l$ | SD | Increase [%] | CV [%] |
|---|---|---|---|---|
| 30.0 | 408860 | 33415 | 100.9 | 8.2 |
| 20.0 | 425787 | 22297 | 107.2 | 5.2 |
| 13.3 | 413606 | 23318 | 102.7 | 5.6 |
| 8.9 | 329913 | 29846 | 71.3 | 9.0 |
| 5.9 | 229459 | 1911 | 33.6 | 0.8 |
| 4.0 | 182477 | 12599 | 15.9 | 6.9 |
| 2.6 | 155997 | 8868 | 6.0 | 5.7 |
| 1.8 | 150951 | 16017 | 4.1 | 10.6 |
| | | | IC50: | 7.16 |

Figure 5 :

Figure 6 :

**NCX1 Inhibition**

Calculation: Sum 50 to 90 seconds.

| Sub 1: | A000135933 | Control: | MW | SD | CV | z´ | S/B |
|--------|------------|----------|-----|-----|-----|-----|-----|
| | | Low | 89159 | 6571 | 7.37 | 0.687743 | 4.5 |
| Sub 2: | A000136648 | | | | | | |
| | | High | 400289 | 25813 | 6.45 | | |
| Sub 3: | A000103746 | File = E:\Flipr\Data\2004_09\09092004_n3.fwd | | | | | |
| Sub 4: | A000104243 | | | | | | |

| | A000135933 | | | |
|------|--------|-------|--------|-------|
| 30.0 | 401905 | 46360 | 100.52 | 11.54 |
| 20.0 | 389304 | 41145 | 96.47 | 10.57 |
| 13.3 | 373523 | 22746 | 91.40 | 6.09 |
| 8.9 | 336706 | 39464 | 79.56 | 11.72 |
| 5.9 | 259225 | 23273 | 54.66 | 8.98 |
| 4.0 | 191173 | 10585 | 32.79 | 5.54 |
| 2.6 | 128657 | 4291 | 12.70 | 3.34 |
| 1.8 | 118381 | 6167 | 9.39 | 5.21 |
| | IC50: | #Ok | | 5.63 |

A000135933 — Fit

| | A000136648 | | | |
|------|--------|-------|-------|-------|
| 30.0 | 295688 | 27743 | 66.38 | 9.38 |
| 20.0 | 232222 | 544 | 45.98 | 0.23 |
| 13.3 | 186038 | 3743 | 31.14 | 2.01 |
| 8.9 | 154215 | 5593 | 20.91 | 3.63 |
| 5.9 | 120528 | 15315 | 10.08 | 12.71 |
| 4.0 | 106876 | 7589 | 5.69 | 7.10 |
| 2.6 | 87441 | 12404 | -0.55 | 14.19 |
| 1.8 | 99043 | 3773 | 3.18 | 3.81 |
| | IC50: | #Ok | | 20.87 |

A000136648 — Fit

| | A000103746 | | | |
|------|-------|------|-------|------|
| 30.0 | 72688 | 6122 | -5.29 | 8.42 |
| 20.0 | 72729 | 2131 | -5.28 | 2.93 |
| 13.3 | 69896 | 6540 | -6.19 | 9.36 |
| 8.9 | 74412 | 3258 | -4.74 | 4.38 |
| 5.9 | 73090 | 2003 | -5.16 | 2.74 |
| 4.0 | 76463 | 2442 | -4.08 | 3.19 |
| 2.6 | 79071 | 2287 | -3.24 | 2.89 |
| 1.8 | 89704 | 4823 | 0.18 | 5.38 |

A000103746 — Fit

A000104243 — Fit

| | | | IC50: | #Ok | >30 | |
|---|---|---|---|---|---|---|

| | A000104243 | | | | |
|---|---|---|---|---|---|
| 30.0 | 363536 | 40034 | 88.19 | 11.01 | #Ok |
| 20.0 | 249177 | 20375 | 51.43 | 8.18 | |
| 13.3 | 199235 | 3502 | 35.38 | 1.76 | |
| 8.9 | 154611 | 973 | 21.04 | 0.63 | |
| 5.9 | 133285 | 2177 | 14.18 | 1.63 | |
| 4.0 | 119586 | 1172 | 9.78 | 0.98 | |
| 2.6 | 108782 | 13627 | 6.31 | 12.53 | |
| 1.8 | 98781 | 10012 | 3.09 | 10.14 | |
| | | IC50: | #Ok | | 18.40 |

Figure 7 :

**European Patent Office**

## EUROPEAN SEARCH REPORT

Application Number

EP 08 29 0264

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FANG Y ET AL: "Na+-Ca2+ exchange and Ca2+ efflux in transfected Chinese hamster ovary cells" July 1999 (1999-07), CELL CALCIUM, VOL. 26, NR. 1-2, PAGE(S) 15-24 , XP002488828 ISSN: 0143-4160 * abstract * * page 16, left-hand column, paragraph 4 - right-hand column, paragraph 1 * * page 16, right-hand column, paragraph 3 - page 17, left-hand column, paragraph 3 * * page 18, left-hand column, paragraph 2 - page 21, left-hand column, paragraph 1 * * figures 3,5 * | 1-6,8, 10,11, 13-18, 22,23 | INV. G01N33/68 |
| Y | * page 16, right-hand column, paragraph 3 - page 17, left-hand column, paragraph 3 * | 7,9,12, 21,24-29 | |
| X | TASHIRO M ET AL: "Transport of magnesium by two isoforms of the Na+-Ca2+ exchanger expressed in CCL39 fibroblasts." October 2000 (2000-10), PFLÜGERS ARCHIV : EUROPEAN JOURNAL OF PHYSIOLOGY OCT 2000, VOL. 440, NR. 6, PAGE(S) 819 - 827 , XP002488829 ISSN: 0031-6768 * page 820, left-hand column, paragraph 3 - right-hand column, paragraph 4 * * page 822, right-hand column, paragraph 1 - paragraph 2 * * figure 3 * | 1-6,8, 10,11, 13-18, 22,23 | TECHNICAL FIELDS SEARCHED (IPC) G01N |
| Y | * page 820, left-hand column, paragraph 3 - right-hand column, paragraph 4 * | 7,9,12, 21,24-29 | |

-/--

The present search report has been drawn up for all claims

| Place of search | Date of completion of the search | Examiner |
|---|---|---|
| The Hague | 21 July 2008 | Chrétien, Eva Maria |

EPO FORM 1503 03.82 (P04C01)

## EUROPEAN SEARCH REPORT

**European Patent Office**

Application Number

EP 08 29 0264

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | RESENDES M C ET AL: "Nitrous oxide enhances Na+/Ca++ exchange in the neuroblastoma cell line SK-N-SH." February 1997 (1997-02), THE JOURNAL OF PHARMACOLOGY AND EXPERIMENTAL THERAPEUTICS FEB 1997, VOL. 280, NR. 2, PAGE(S) 795 - 801 , XP002488830 ISSN: 0022-3565 * page 795, right-hand column, paragraph 3 - page 796, left-hand column, paragraph 3 * * page 797, right-hand column, paragraph 1 * * figures 3,4 * | 1-6,8, 10,11, 13-18, 22,23 | |
| X | HUNTON DACIA L ET AL: "Adult rat cardiomyocytes exhibit capacitative calcium entry." March 2004 (2004-03), AMERICAN JOURNAL OF PHYSIOLOGY. HEART AND CIRCULATORY PHYSIOLOGY MAR 2004, VOL. 286, NR. 3, PAGE(S) H1124 - H1132 , XP002488831 ISSN: 0363-6135 * page H1125, left-hand column, paragraph 3 * * page H1126, left-hand column, paragraph 2 - right-hand column, paragraph 1 * * figure 3 * | 1-6,8, 10,11, 13-18, 22,23 | TECHNICAL FIELDS SEARCHED (IPC) |

-/--

The present search report has been drawn up for all claims

| Place of search | Date of completion of the search | Examiner |
|---|---|---|
| The Hague | 21 July 2008 | Chrétien, Eva Maria |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

## EUROPEAN SEARCH REPORT

Application Number

EP 08 29 0264

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | NAMEKATA ET AL: "Reduction by SEA0400 of myocardial ischemia-induced cytoplasmic and mitochondrial Ca<2+> overload" EUROPEAN JOURNAL OF PHARMACOLOGY, AMSTERDAM, NL, vol. 543, no. 1-3, 14 August 2006 (2006-08-14), pages 108-115, XP005573423 ISSN: 0014-2999 * page 109, right-hand column, paragraph 3 - page 110, left-hand column, paragraph 2 * | 1-6,8, 10,11, 13-18, 22,23 | |
| Y | EP 1 241 260 A (MILLENIUM PHARMACEUTICALS INC [US]) 18 September 2002 (2002-09-18) * paragraphs [0018], [0053], [0274] * | 12,24-29 | |
| Y | WO 02/31508 A (SQUIBB BRISTOL MYERS CO [US]; WEAVER CHARLES DAVID [US]) 18 April 2002 (2002-04-18) * page 5, line 12 - line 22 * * page 16, line 20 - page 17, line 5 * * page 21, line 25 - page 22, line 4 * * page 25, line 16 - page 26, line 5 * | 7,9,21 | |
| A | EP 1 526 140 A (YAMANOUCHI PHARMA CO LTD [JP] ASTELLAS PHARMA INC [JP]) 27 April 2005 (2005-04-27) * the whole document * | 1-29 | |

TECHNICAL FIELDS SEARCHED (IPC)

-/--

The present search report has been drawn up for all claims

| Place of search | Date of completion of the search | Examiner |
|---|---|---|
| The Hague | 21 July 2008 | Chrétien, Eva Maria |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

........................................................................................
& : member of the same patent family, corresponding
   document

EPO FORM 1503 03.82 (P04C01)

European Patent Office

**EUROPEAN SEARCH REPORT**

Application Number

EP 08 29 0264

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MASUO ET AL: "Hippocalcin protects hippocampal neurons against excitotoxin damage by enhancing calcium extrusion" NEUROSCIENCE, NEW YORK, NY, US, vol. 145, no. 2, 7 March 2007 (2007-03-07), pages 495-504, XP022028114 ISSN: 0306-4522 * the whole document * | 1-29 | |
| A | SECONDO ET AL: "BHK cells transfected with NCX3 are more resistant to hypoxia followed by reoxygenation than those transfected with NCX1 and NCX2: Possible relationship with mitochondrial membrane potential" CELL CALCIUM (EDINBURGH), CHURCHILL LIVINGSTONE MEDICAL JOURNALS, EDINBURGH, GB, vol. 42, no. 6, 29 September 2007 (2007-09-29), pages 521-535, XP022271638 ISSN: 0143-4160 * the whole document * | 1-29 | |
| | | | **TECHNICAL FIELDS SEARCHED (IPC)** |

The present search report has been drawn up for all claims

| Place of search | Date of completion of the search | Examiner |
|---|---|---|
| The Hague | 21 July 2008 | Chrétien, Eva Maria |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

      .................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 08 29 0264

This annex lists the patent family members relating to the patent documents cited in the above-mentioned European search report.
The members are as contained in the European Patent Office EDP file on
The European Patent Office is in no way liable for these particulars which are merely given for the purpose of information.

21-07-2008

| Patent document cited in search report | | Publication date | Patent family member(s) | | Publication date |
|---|---|---|---|---|---|
| EP 1241260 | A | 18-09-2002 | NONE | | |
| WO 0231508 | A | 18-04-2002 | AU | 1535002 A | 22-04-2002 |
| | | | AU | 2002215350 B2 | 07-12-2006 |
| | | | CA | 2425806 A1 | 18-04-2002 |
| | | | EP | 1327150 A1 | 16-07-2003 |
| | | | JP | 2004530100 T | 30-09-2004 |
| | | | MX | PA03003223 A | 10-09-2003 |
| EP 1526140 | A | 27-04-2005 | AU | 2003252754 A1 | 23-02-2004 |
| | | | CA | 2480427 A1 | 12-02-2004 |
| | | | WO | 2004013173 A1 | 12-02-2004 |
| | | | US | 2005119173 A1 | 02-06-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1031556 A
- US 5714666 A

**Non-patent literature cited in the description**

- **Ohtsuka.** *Journal of Pharmacological. Sciences.,* 2004
- **Hobai, JA ; O'Rourke,B.** *Expert Opin. Investig. Drugs,* 2004, vol. 13, 653-664
- **Iwamoto, T. et al.** *J. Biol. Chem.,* 2004, vol. 279, 7544-7553
- **Hinata, M. et al.** *J. Physiol.,* 2002, vol. 545, 453-461
- **C. Lee et al.** *The journal of pharmacology and experimental therapeutics,* 2004, vol. 311, 748-757
- **Tong Mook Kang ; Donald W. Hilgemann.** *Nature,* 05 February 2004, vol. 427
- **T. Kuramochi et al.** *Bioorganic & Medicinal Chemistry,* 2004, vol. 12, 5039-5056
- **Nicoll, DA. et al.** *Science,* 1990, vol. 250 (4980), 562-5
- **Komuro, I. et al.** *Proc. Natl. Acad. Sci. U.S.A.,* 1992, vol. 89 (10), 4769-4773
- **Kofuji, P. et al.** *Am. J. Physiol.,* 263
- *Cell Physiol.,* 1992, vol. 32, C1241-C1249
- **Li, Z. et al.** *J. Biol. Chem.,* 1994, vol. 269 (26), 17434-9
- **Nicoll, DA.** *J. Biol. Chem.,* 1996, vol. 271 (40), 24914-21
- **Gabellini, N.** *Gene,* 2002, vol. 298, 1-7
- Current protocols in cell biology. John Wiley & Sons Inc
- **Créton et al.** *Microscopy Research and Technique,* 1999, vol. 46, 390-397
- **Brini et al.** *J. Biol. Chem.,* 1995, vol. 270, 9896-9903
- **Knight ; Knight.** *Meth. Cell. Biol.,* 1995, vol. 49, 201-216
- Current protocols in molecular biology. John Wiley & Sons Inc